# EUROPEAN PATENT SPECIFICATION

(11) **EP 2 170 297 B1**
(45) Date of publication and mention of the grant of the patent: **08.08.2012**
(21) Application number: 08762415.1
(22) Date of filing: 20.06.2008
(51) Int. Cl.: A61K 9/36, A61K 9/62, A61K 31/381, A61P 25/24

(54) **DULOXETINE FORMULATION**
FORMULIERUNG VON DULOXETIN
FORMULATION DE DULOXÉTINE

(30) Priority: 23.06.2007 GB 0712220; 07.03.2008 GB 0804270
(43) Date of publication of application: 07.04.2010
(73) Proprietor: Arrow International Limited, Valetta, VLT 08 (MT)
(72) Inventor: SAREEN, Rahul, Croydon South, VIC 3136 (AU); PERSICANER, Peter, Henry, Robert, Croydon South, VIC 3136 (AU)
(74) Representative: Schlich, George William
(86) International application number: PCT/GB2008/002098
(87) International publication number: WO 2009/001043

(56) References cited:
- WO-A-2007/034503
- US-A1- 2006 165 776

## Description

The present invention relates to pharmaceutical formulations of the active compound duloxetine and to processes for preparing the formulations.

Duloxetine (+)-(*S*)-*N*-methyl-y-(1-naphthyloxy)-2-thiophenepropylamine hydrochloride is a well known drug within the class known as SSNRI (selective serotonin and norepinephrine reuptake inhibitor) compounds, which are indicated for use in the treatment of major depressive disorder (MDD), diabetic peripheral neuropathic pain and incontinence (specifically stress urinary incontinence (SUI)). It is typically used in the form of its hydrochloride salt and use of the term "duloxetine" hereinafter is intended to refer generally to the free base but also to salts and other compounds of duloxetine, though typically to the hydrochloride salt.

It is known that duloxetine is unstable in acidic media. Accordingly, it is desired to protect the active compound from the acidic conditions of the stomach and allow it to be released in the near neutral conditions of the gastrointestinal (GI) tract, such as in the small intestine. Typically active compounds which are acid-sensitive are administered as an enterically coated formulation, wherein the enteric coating protects the active compound from the acidic conditions of the stomach and releases the active in a near neutral environment such as in the small intestine.

However, it is also known that duloxetine reacts with many known enteric coatings to form a slowly soluble or even insoluble coating which decreases the bioavailability of the active compound.

US 5,508,276 discloses duloxetine enteric pellets in which hydroxypropylmethylcellulose acetate succinate (hereinafter HPMCAS) is used as an enteric coating. In order to reduce the reaction between the duloxetine active and the acidic groups of the HPMCAS, the HPMCAS is at least partly neutralised with ammonia prior to being used in the coating mixture for the pellets. It is stated that the polymer used in the enteric coating layer must be selected to have only a small number of carboxylic acid groups per unit weight or repeating unit of the polymer. This is the reason for selecting HPMCAS as the enteric polymer, as it has between 4 and 28% of succinoyl groups.

US 2006/165776 describes duloxetine-containing formulations, and discloses that duloxetine reacts with polymer degradation products or residual free acids present in HPMCAS and HPMCP polymers used as enteric coatings. US 2006/0165776 avoids such problems by providing a formulation that does not comprise any alkaline reacting compounds or other buffering agents.

According to a first aspect of the present invention, a duloxetine formulation is provided, comprising (i) a core including a desired amount of duloxetine and (ii) an enteric coating comprising hydroxypropylmethylcellulose phthalate (HPMCP). Hence HPMCP is used as an enteric polymer. The formulation also comprises (iii) a separating layer located between the core and the enteric coating, the separating layer including polyvinyl alcohol. The formulation is preferably in the form of a pellet, but can also be in the form of a tablet, comprising a duloxetine core coated with an enteric coating comprising HPMCP as enteric polymer.

The formulations of the invention are found to offer comparable or improved stability compared to known formulations.

In particular formulations of the invention, through the use of the separating layer, it has been found that it is possible to use an alternative enteric coating, namely comprising hydroxypropylmethylcellulose phthalate (hereinafter HPMCP), to provide acceptable stability and bioavailability, and without the need to neutralise at least some of the carboxylic acid groups. The HPMCP in the enteric coating may constitute 10% or more of the total formulation weight.

In addition to reducing or preventing reaction between the duloxetine and the HPMCP of the enteric coating, the separating layer polymer typically is also itself unreactive with both the duloxetine active and the HPMCP.

In an embodiment of the invention, the separating layer further includes a cellulose polymer; this may for example be a hydroxypropylmethylcellulose (HPMC) polymer, optionally a low molecular weight HPMC polymer.

In a further embodiment of the invention, the separating layer comprises a first layer comprising a low molecular weight HPMC polymer and a second layer comprising polyvinyl alcohol.

The separating layer may represent 2 to 20% by weight of the pellet formulation, optionally 5 to 15% by weight, further optionally 7 to 12% by weight.

In an embodiment of the invention, the separating layer may contain 15 to 45% by weight (of the separating layer) of a low molecular weight HPMC layer and 10 to 38% by weight of polyvinyl alcohol in a separate polyvinyl alcohol layer. Optionally, the separating layer contains 20 to 40% by weight of the HPMC layer and 16 to 32% by weight of polyvinyl alcohol, further optionally 25 to 35% by weight of the HPMC layer and 20 to 28% by weight of polyvinyl alcohol. The separating layer may also contain up to 60% by weight (optionally up to 45% by weight) of additional pharmaceutical excipients, such as for example, glidants.

The core of each pellet may comprise a pharmaceutically acceptable inert bead upon which is coated the duloxetine active. Such inert beads are well known to those skilled in the art and include beads made from sugar and/or starch, as well as from vegetable gums, microcrystalline cellulose, waxes, etc. Suitable beads having the desired physical characteristics and size range are typically commercially available.

When used in the preparation of duloxetine pellet formulations, the bead is suitably selected so as not to react with the duloxetine. Thus, it should preferably be substantially free from acid moieties or impurities. Sugar spheres have been found to form suitable beads upon which the duloxetine may be carried. Other bead components may also be suitable.

A suitable size range for the inert beads is 15 to 50 mesh, optionally 20 to 40 mesh. In certain embodiments of the invention, the size range of the inert beads is 25 to 30 mesh.

Optionally the core may further include additional pharmaceutical excipients typically used in multi-layer bead formulations, for example a binder.

The core may comprise 50 to 90% by weight of the pellet, optionally 60 to 80% by weight, further optionally 65 to 75% by weight.

In addition, the inert bead may comprise 50 to 90% by weight of the core and the duloxetine active may comprise 10 to 50% by weight of the core. In an embodiment of the invention, the inert bead may comprise 60 to 80% by weight of the core and the duloxetine may comprise 20 - 40% by weight of the core. In a further embodiment of the invention, the core may comprise 65 to 75% by weight inert bead and 25 to 35% by weight duloxetine. The core may also include up to 5% by weight (optionally up to 1% by weight) of additional excipient components, such as a binder.

The enteric coating may contain conventional pharmaceutical excipients in addition to the HPMCP enteric polymer. These may include glidants and plasticizers. Thus, the enteric coating may comprise 70 to 100% by weight (of the coating) of HPMCP polymer, optionally 75 to 90% by weight. The enteric coating may comprise up to 30% by weight of additional pharmaceutically acceptable excipients, optionally up to 25% by weight.

In an embodiment of the invention, the duloxetine pellet formulation comprises:
65-75% by weight of the duloxetine-containing core,
5 to 15% by weight of the separating layer, and
15 to 25% by weight of the enteric coating.

In a further embodiment of the invention, the duloxetine pellet formulation comprises:
65-75% by weight of the duloxetine-containing core,
2-5% by weight HPMC separating layer,
3-10% by weight polyvinyl alcohol separating layer, and
15 to 25% by weight of the enteric coating.

In a still further embodiment of the invention, the duloxetine pellet formulation comprises:
45-55% by weight of an inert bead,
15-25% by weight duloxetine active (as hydrochloride salt),
2-5% by weight HPMC separating layer,
3-10% by weight polyvinyl alcohol separating layer, and
10-20% by weight HPMCP enteric polymer.

It will be understood by those skilled in the art that the above percentages by weight in respect of the pellet formulations refer to the total weight of the pellet. The polyvinyl alcohol separating layer typically contains about 30 - 60% polyvinyl alcohol, hence in the above formulations there is typically from about 1 to about 5% polyvinyl alcohol.

In order to deliver the desired dose of duloxetine to a patient, typically a mammalian patient, for example a human patient, the pellets may be dispensed via a capsule, wherein the capsule contains sufficient number or weight of pellets to deliver the desired dose of duloxetine to the patient. Thus, a second aspect of the invention provides a capsule comprising a number of pellets as defined above in any embodiment sufficient to provide a pharmaceutically active dose of duloxetine to a patient.

The majority of capsules used in the pharmaceutical industry are made from gelatine - i.e. the capsule shell is or comprises gelatine. However, hard gelatine capsules tend to have a relatively high moisture content (typically 13-16%w/w) and this may result in a decrease in stability of the pellets. In an embodiment of the invention, the capsule is an HPMC capsule. HPMC capsule shells used herein have a lower moisture content, generally 10% w/w or less, typically 4-6% w/w, compared with gelatine capsules and are therefore less likely to affect the stability of the pellets therein. Preferred capsule shells of the invention have a moisture content of 10% w/w or less, and more preferred capsules comprise or consist essentially of HPMC.

Optionally, the formulation of the invention may comprise a glidant to aid encapsulation of the finished enteric coated pellets in capsules. Thus, the encapsulation aid may comprise 0.1 to 10% by weight of the pellet formulation, optionally 0.2 to 5% by weight, further optionally 0.3 to 1% by weight.

The duloxetine formulation is generally in a delayed release dosage form, preferably having an equivalent bioavailability to known Brand delayed release duloxetine formulations. Accordingly, in an embodiment of the invention the dosage form may have a dissolution rate such that the formulation is completely dissolved within 4 hours, optionally the formulation is completely dissolved within 2 hours and further optionally the formulation is completely dissolved within 1.5 hours once transferred into buffer medium, when tested according to FDA dissolution methodology comprising: [A] gastric challenge: 0.1N HCl for 2 hours; [B] buffer medium: pH 6.8 phosphate buffer (USP) 15, 30, 45, 60 and 90 minutes.

According to a third aspect of the invention, there is provided the use of duloxetine, polyvinyl alcohol and hydroxypropylmethylcellulose phthalate in the manufacture of a medicament for the treatment of conditions for which SSNRI compounds are indicated in a patient, wherein the duloxetine forms part of a core, the hydroxypropylmethylcellulose phthalate forms part of an enteric coating and the polyvinyl alcohol forms a separating layer which separates the core from the enteric coating.

In accordance with the foregoing, the separating layer may further include a cellulose polymer. In an embodiment of this aspect of the invention, the separating layer comprises a first layer including a low molecular weight hydroxypropylmethylcellulose polymer and a second layer including polyvinyl alcohol.

As mentioned above, the patient may be a mammalian patient, optionally a human patient.

According to a fourth aspect of the invention, there is provided a process for preparing a duloxetine pellet formulation as defined anywhere above, the process comprising:
(i) providing a core including a desired amount of duloxetine;
(ii) applying a separating layer including polyvinyl alcohol to the core; and
(iii) applying an enteric coating comprising hydroxypropylmethylcellulose phthalate to the separating layer.

The preparation of a drug-containing core for use in a multi-layer pellet formulation is well known in the art. More specifically, the preparation of duloxetine-containing cores are disclosed and discussed in US 5,508,276, the content of which is incorporated herein by reference.

In embodiments in which the separating layer comprises two separate layers, one including HPMC and the other including polyvinyl alcohol, Step (ii) above may comprise the sub-steps:
(a) applying a first polymer layer comprising a low molecular weight hydroxypropylmethylcellulose; and
(b) applying a second polymer layer comprising polyvinyl alcohol.

Steps (i), (ii) and/or (iii) above may be carried out in a fluid bed apparatus. In addition, in embodiments where step (ii) comprises the sub-steps (a) and (b), both of the sub-steps may be carried out in a fluid bed apparatus. In an embodiment of the invention, all of the steps (i), (ii) and (iii) are carried out in a fluid bed apparatus, optionally the same fluid bed apparatus, i.e. without removing the intermediate products between steps.

The various embodiments and features of the invention as defined above in any aspect or embodiment of the invention may be combined with one or more other embodiments or features of the invention unless expressly stated otherwise. In particular, the skilled person will appreciate that features defined in respect of one aspect of the invention will be equally applicable to other aspects of the invention and are not intended to be limited to just one aspect of the invention unless expressly stated otherwise or the context limits the relevance to a single aspect or specified aspects of the invention.

The following Examples set out the preparation of a number of duloxetine pellets and capsule formulations.

### Formulations:

| **EXAMPLE** | **1** | **2** | **3** | **4** |
|---|---|---|---|---|
| **Ingredient** | **Weight (mg)** | **Weight (mg)** | **Weight (mg)** | **Weight (mg)** |
| **Core** | | | | |
| Sugar Spheres 25-30 mesh | 168.000 | 112.000 | 84.000 | 56.000 |
| Duloxetine as HCl | 67.300 | 44.867 | 33.650 | 22.433 |
| (equivalent to duloxetine base) | (60.000) | (40.000) | (30.000) | (20.000) |
| Povidone K 30 | 1.700 | 1.133 | 0.850 | 0.567 |
| Purified Water | qs | qs | qs | qs |
| Ethanol 95% | qs | qs | qs | qs |

| **Separating layer** | | | | |
|---|---|---|---|---|
| Opadry Clear OY-29020* | 9.750 | 6.500 | 4.875 | 3.250 |
| Purified Talc (Extra Fine) | 5.250 | 3.500 | 2.625 | 1.750 |
| Purified Water | qs | qs | qs | qs |
| Opadry AMB OY-B-28920 White** | 16.800 | 11.200 | 8.400 | 5.600 |
| Purified Water | qs | qs | qs | qs |

| **Enteric coating** | | | | |
|---|---|---|---|---|
| Hypromellose Phthalate (HPMCP) | 56.000 | 37.333 | 28.00 | 18.667 |
| Purified Talc (Extra Fine) | 5.600 | 3.733 | 2.800 | 1.867 |
| Triethyl Citrate | 5.600 | 3.733 | 2.800 | 1.867 |
| Ethanol 95% | qs | qs | qs | qs |
| Purified Water | qs | qs | qs | qs |
| **TOTAL FILL WEIGHT** | **336.000** | **224.000** | **168.000** | **112.000** |
| **Hard Gelatin or HPMC Capsule Size** | **"1"** | **"2"** | **"3"** | **"4"** |

| | | | | |
|---|---|---|---|---|
| *Components consist of Hypromellose 6cP 90.91% & Polyethylene Glycol 400 9.09%. **Components consist of Polyvinyl Alcohol (part hydrolysed polyvinyl acetate) 45.52%, Titanium Dioxide 32.00%, Talc 20.00%, Lecithin (Soya) 2.00% & Xanthan Gum 0.48%). | | | | |

Further duloxetine pellet and capsule formulations were prepared as follows:-

| **EXAMPLE** | **5** | **6** | **7** | **8** |
|---|---|---|---|---|
| **STRENGTH (as base in mg)** | **60.0 mg** | **40.0 mg** | **30.0 mg** | **20.0 mg** |

| **Ingredient** | **Weight (mg)** | **Weight (mg)** | **Weight (mg)** | **Weight (mg)** |
|---|---|---|---|---|
| Sugar Spheres 25-30 mesh | 168.000 | 112.000 | 84.000 | 56.000 |

| **CORE** | | | | |
|---|---|---|---|---|
| Duloxetine as HCl | 67.300 | 44.867 | 33.650 | 22.433 |
| Povidone K 30 | 1.700 | 1.133 | 0.850 | 0.567 |
| Purified Water | qs | qs | qs | qs |
| Ethanol 95% | qs | qs | qs | qs |

| **SEPARATING LAYER** | | | | |
|---|---|---|---|---|
| Opadry Clear OY-29020* | 9.750 | 6.500 | 4.875 | 3.250 |
| Purified Talc (Extra Fine) | 5.250 | 3.500 | 2.625 | 1.750 |
| Purified Water | qs | qs | qs | qs |
| Opadry AMB OY-B-28920 White** | 20.000 | 13.333 | 10.000 | 6.667 |
| Purified Water | qs | qs | qs | qs |

| **ENTERIC COAT** | | | | |
|---|---|---|---|---|
| Hypromellose Phthalate (HPMCP) | 53.336 | 35.557 | 26.668 | 17.779 |
| Purified Talc (Extra Fine) | 5.332 | 3.555 | 2.666 | 1.777 |
| Triethyl Citrate | 5.332 | 3.555 | 2.666 | 1.777 |
| Ethanol 95% | qs | qs | qs | qs |
| Purified Water | qs | qs | qs | qs |

| **LUBRICATION** | | | | |
|---|---|---|---|---|
| Purified Talc | 2.000 | 1.333 | 1.000 | 0.667 |

| **ENCAPSULATION** | | | | |
|---|---|---|---|---|
| **Hard Gelatin or HPMC Capsule Size** | "1" | "2" | "3" | "4" |
| **TOTAL FILL WEIGHT** | **338.000** | **225.333** | **169.000** | **112.667** |

| | | | | |
|---|---|---|---|---|
| *Components consist of Hypromellose 6cP & Polyethylene Glycol 400 **Components consist of Polyvinyl alcohol-part. Hydrolysed, Titanium Dioxide, Talc, Lecithin (Soya) & Xanthan Gum | | | | |

### Preparation

The formulations of examples 1-8 were prepared as follows:-

### Step 1: Preparation of the core

The duloxetine HCl and Povidone K-30 (binder) were dissolved in the ethanol 95% and purified water. This solution was coated on the sugar spheres in a fluid bed Wurster coater.

### Step 2: The separating layer

The drug-coated spheres were then coated in the fluid bed Wurster coater firstly with a dispersion of Opadry Clear OY-29020 and purified talc (glidant) in purified water and then secondly with a dispersion of Opadry AMB OY-B-28920 (containing PVA) in purified water to provide seal-coated particles.

### Step 3: The enteric coating

The seal-coated particles were then coated in the fluid bed Wurster coater with a dispersion of hydroxypropylmethylcellulose phthalate (HPMCP), purified talc (glidant) and triethyl citrate (plasticizer) in purified water and ethanol 95% to provide the enterically coated duloxetine pellets.

All of the above steps were carried out using conventional conditions and techniques for the Wurster coating process.

All of the above example formulations were found to have acceptable stability and dissolution profiles.

The skilled person will appreciate that variations in the process and the ingredients can be made in accordance with their common general knowledge. For example, different coating techniques and apparatus could be used and/or different excipients could be included in the formulations.

### Example 9

The following study was carried out to demonstrate the release profile of a formulation of the invention (FDH0602) in comparison with a known market brand (Cymbalta, A2523966) and the controlled release formulations of duloxetine disclosed in WO 2007/034503 (Ex. 1-8 below).

A2523966 and FDH0602 capsules (delayed release pellets) were tested according to FDA dissolution methodology:
Apparatus I @ 100 rpm
[A] Gastric Challenge: 0.1 N HCl for 2 hours
[B] Buffer Medium: pH 6.8 phosphate buffer (USP) 15, 30, 45, 60 and 90 minutes

The dissolution data for Ex. 1-8 below were obtained from the specification of WO 2007/034503.

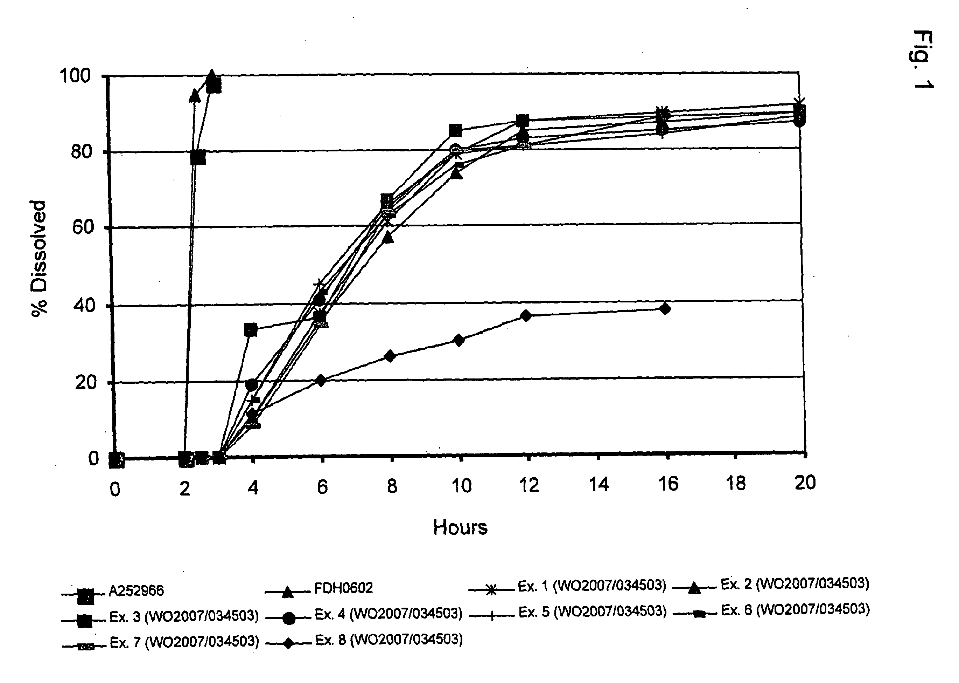

The dissolution data for all samples can be seen in the table below and in figure 1.

| **Formulation** | **A2523966** | **FDH0602** | **Ex. 1** | **Ex. 2** | **Ex. 3** | **Ex. 4** | **Ex. 5** | **Ex. 6** | **Ex. 7** | **Ex. 8** |
|---|---|---|---|---|---|---|---|---|---|---|
| **Time (hrs)** | **% Dissolved** | | | | | | | | | |
| **0** | 0 | 0 | 0 | 0 | 0 | 0 | 0 | 0 | 0 | 0 |
| **0.5** | 0 | 0 | 0 | 0 | 0 | 0 | 0 | 0 | 0 | 0 |
| **1** | 0 | 0 | 0 | 0 | 0 | 0 | 0 | 0 | 0 | 0 |
| **2** | 0 | 0 | 0 | 0 | 0 | 0 | 0 | 0 | 0 | 0 |
| **2.5** | 79 | 95 | Nd | Nd | Nd | Nd | Nd | Nd | Nd | Nd |
| **3** | 98 | 100 | Nd | Nd | Nd | Nd | Nd | Nd | Nd | Nd |
| **4** | | | 10 | 10 | 33 | 19 | 15 | 15 | 8 | 11 |
| **6** | | | 37 | 35 | 36 | 41 | 45 | 43 | 34 | 20 |
| **8** | | | 61 | 57 | 67 | 65 | 66 | 63 | 64 | 26 |
| **10** | | | 79 | 74 | 85 | 80 | 79 | 76 | 80 | 30 |
| **12** | | | 88 | 85 | 88 | 83 | 81 | 81 | 81 | 36 |
| **16** | | | 90 | 87 | 89 | 85 | 84 | 89 | 85 | 38 |
| **20** | | | 92 | 90 | 90 | 87 | 89 | | | |

| | | | | | | | | | | |
|---|---|---|---|---|---|---|---|---|---|---|
| **Nd = Not determined** | | | | | | | | | | |

The formulation of the present invention (FDH0602) is completely dissolved after less than 1 hour in the buffer medium, demonstrating similar *in vitro* release to the known brand product (Cymbalta, A2523966). This contrasts with the controlled release formulations of WO 2007/034503 which show less than 35% release (often significantly less) after 2 hours in the buffer medium and incomplete dissolution after 16 hours.

The invention thus provides duloxetine formulations, capsules and use thereof and processes for the preparation of duloxetine formulations.

## Claims

1. A duloxetine formulation comprising: (i) a core including an amount of duloxetine; (ii) an enteric coating comprising hydroxypropylmethylcellulose phthalate, and (iii) a separating layer located between the core and the enteric coating, wherein the separating layer comprises polyvinyl alcohol plus, optionally, a cellulose polymer.

2. A duloxetine formulation according to claim 1, wherein the separating layer comprises a first layer including a low molecular weight hydroxypropylmethylcellulose polymer and a second layer including polyvinyl alcohol.

3. A duloxetine formulation according to claim 1 or 2, wherein the core comprises a pharmaceutically acceptable inert bead which carries the duloxetine and a pharmaceutically acceptable binder.

4. A duloxetine formulation according to any of claims 1-3, in the form of a pellet.

5. A duloxetine formulation according to any of claims 1-3, in the form of a tablet.

6. A capsule comprising a plurality of pellets according to claim 4.

7. A capsule according to claim 6, having a capsule shell, wherein the capsule shell has a moisture content of 10% w/w or less.

8. A duloxetine formulation according to any of claims 1-7, wherein the formulation is in a delayed release dosage form.

9. A duloxetine formulation according to claim 8, wherein the dosage form is dissolved in less than 1.5 hours once transferred into buffer medium when tested according to FDA dissolution methodology comprising: [A] gastric challenge: 0.1 N HCl for 2 hours; [B] buffer medium: pH 6.8 phosphate buffer (USP) 15, 30, 45, 60 and 90 minutes.

10. Use of duloxetine and hydroxypropylmethylcellulose phthalate in the manufacture of a medicament for the treatment of conditions for which SSNRI compounds are indicated in a patient, wherein the duloxetine forms part of a core and the hydroxypropylmethylcellulose phthalate forms part of an enteric coating, further comprising a separating layer which separates the core from the enteric coating, wherein the separating layer comprises polyvinyl alcohol plus, optionally, a cellulose polymer.

11. Use according to claim 10, wherein the separating layer comprises a first layer including a low molecular weight hydroxypropylmethylcellulose polymer and a second layer including polyvinyl alcohol.

12. A process for preparing a duloxetine formulation according to any of claims 1 to 9, the process comprising:
(i) providing a core including a desired amount of duloxetine;
(ii) applying an enteric coating comprising hydroxypropylmethylcellulose phthalate to the core; and
(iii) applying a separating layer to the core and applying the enteric coating to the separating layer,
wherein the separating layer of (iii) comprises polyvinyl alcohol plus, optionally, a cellulose polymer.

13. A process according to claim 12, wherein step (iii) comprises the sub-steps:
(a) applying a first layer comprising a low molecular weight hydroxypropylmethylcellulose polymer; and
(b) applying a second polymer layer comprising polyvinyl alcohol.

14. A process according to claim 12 or 13, wherein step (ii) and/or step (iii) is/are conducted in a fluid bed apparatus.

15. A process according to any of claims 12-14, wherein step (i) comprises applying the duloxetine and a binder to a pharmaceutically acceptable inert bead.

## Patentansprüche

1. Duloxetinformulierung umfassend: (i) einen eine Menge an Duloxetin beinhaltenden Kern; (ii) eine Hydroxypropylmethylzellulosephthalat enthaltende enterische Beschichtung, und (iii) eine zwischen dem Kern und der enterischen Beschichtung angeordnete Trennschicht, wobei die Trennschicht Polyvinlalkohol sowie, optional, ein Zellulosepolymer enthält.

2. Duloxetinformulierung nach Anspruch 1, wobei die Trennschicht eine erste, ein niedermolekulares Hydroxypropylmethylzellulosepolymer enthaltende Schicht und eine zweite, Polyvinylalkohl enthaltende Schicht, umfasst.

3. Duloxetinformulierung nach Anspruch 1 oder 2, wobei der Kern ein pharmazeutisch annehmbares Inert-Kügelchen umfasst, das das Duloxetin und einen pharmazeutisch annehmbaren Binder enthält.

4. Duloxetinformulierung nach einem der Ansprüche 1-3 in Form eines Pellets.

5. Duloxetinformulierung nach einem der Ansprüche 1-3 in Form einer Tablette

6. Kapsel enthaltend eine Mehrzahl an Pellets nach Anspruch 4.

7. Kapsel nach Anspruch 6 mit einer Kapselhülle, wobei die Kapselhülle einen Feuchtegehalt von 10 % Gewichtsprozent oder weniger aufweist.

8. Duloxetinformulierung nach einem der Ansprüche 1-7, wobei die Formulierung in einer Dosierungsform mit verzögerter Freisetzung ausgeführt ist.

9. Duloxetinformulierung nach Anspruch 8, wobei die Dosierungsform in weniger als 1.5 Stunden aufgelöst wird, nachdem sie in ein Puffermedium überführt wurde im Rahmen eines Testes gemäss FDA-Auflösungsmethodik umfassend: [A] gastrischer Angriff ("gastric challenge"): 0.1 N HCL während 2 Stunden; [B] Puffermedium: pH 6.8 Phosphatpuffer (USP) 15, 30, 45, 60 and 90 Minuten.

10. Verwendung von Duloxetin und Hydroxypropylmethylzellulosephthalat zur Herstellung eines Medikaments zur Behandlung von Zuständen für welche SSNRI-Verbindungen bei einem Patienten angezeigt sind, wobei das Duloxetin einen Teil einer enterischen Beschichtung bildet, weiter umfassend eine Trennschicht, die den Kern von der enterischen Beschichtung trennt, wobei die Trennschicht Polyvinlalkohol sowie, optional, ein Zellulosepolymer enthält.

11. Verwendung nach Anspruch 10, wobei die Trennschicht eine erste, ein niedermolekulares Hydroxypropylmethylzellulosepolymer enthaltende Schicht und eine zweite, Polyvinylalkohl enthaltende Schicht, umfasst.

12. Verfahren zur Zubereitung einer Duloxetin-Formulierung gemäss einem der Ansprüche 1 bis 9, wobei das Verfahren umfasst:
(i) Bereitstellen eines eine gewünschte Menge an Duloxetin beinhaltenden Kerns;
(ii) Applizieren einer Hydroxypropylmethylzellulosephthalat enthaltenden enterischen Beschichtung auf den Kern; und
(iii) Applizieren einer Trennschicht auf den Kern und Applizieren der enterischen Beschichtung auf die Trennschicht;
wobei die Trennschicht von (iii) Polyvinlalkohol sowie, optional, ein Zellulosepolymer enthält.

13. Verfahren nach Anspruch 12, wobei Schritt (iii) folgende Teilschritte umfasst:
(a) Applizieren einer ersten, ein niedermolekulares Hydroxypropylmethylzellulosepolymer enthaltenden Schicht; und
(b) Applizieren einer zweiten, Polyvinylalkohl enthaltende Polymerschicht.

14. Verfahren nach Anspruch 12 oder 13, wobei Schritt (ii) und/oder Schritt (iii) in einem Wirbelschichtapparat ausgeführt wird/werden.

15. Verfahren nach einer der Ansprüche 12-14, wobei Schritt (1) Applizieren des Duloxetins und eines Binders zu einem pharmazeutisch annehmbaren Inert-Kügelchen umfasst.

## Revendications

1. Formulation de duloxétine comprenant : (i) un noyau comprenant une quantité de duloxétine ; (ii) un enrobage entérique comprenant du phtalate d'hydroxypropylméthylcellulose, et (iii) une couche de séparation située entre le noyau et l'enrobage entérique, la couche de séparation comprenant du poly(alcool vinylique) plus, facultativement, un polymère de cellulose.

2. Formulation de duloxétine selon la revendication 1, dans laquelle la couche de séparation comprend une première couche comprenant un polymère d'hydroxypropylméthylcellulose de faible poids moléculaire et une deuxième couche comprenant du poly(alcool vinylique).

3. Formulation de duloxétine selon la revendication 1 ou 2, dans laquelle le noyau comprend une bille inerte pharmaceutiquement acceptable qui comporte la duloxétine et un liant pharmaceutiquement acceptable.

4. Formulation de duloxétine selon l'une quelconque des revendications 1 à 3, sous la forme d'une pastille.

5. Formulation de duloxétine selon l'une quelconque des revendications 1 à 3, sous la forme d'un comprimé.

6. Capsule comprenant une pluralité de pastilles selon la revendication 4.

7. Capsule selon la revendication 6, ayant une coque de capsule, dans laquelle la coque de capsule a une teneur en humidité de 10 % m/m ou moins.

8. Formulation de duloxétine selon l'une quelconque des revendications 1 à 7, dans laquelle la formulation est sous une forme pharmaceutique à libération retardée.

9. Formulation de duloxétine selon la revendication 8, dans laquelle la forme pharmaceutique est dissoute en moins de 1,5 heure une fois transféré dans un milieu tampon lorsqu'elle est soumise à essai selon la méthodologie de dissolution de la FDA comprenant : [A] l'épreuve gastrique : HCl 0,1 N pendant 2 heures ; [B] un milieu tampon : tampon phosphate pH 6,8 (USP) 15, 30, 45, 60 et 90 minutes.

10. Utilisation de duloxétine et de phtalate d'hydroxypropylméthylcellulose dans la fabrication d'un médicament pour le traitement d'affections pour lesquelles des composés SSNRI sont indiqués chez un patient, la duloxétine faisant partie d'un noyau et le phtalate d'hydroxypropylméthylcellulose faisant partie d'un enrobage entérique, comprenant en outre une couche de séparation qui sépare le noyau de l'enrobage entérique, la couche de séparation comprenant du poly(alcool vinylique) plus, facultativement, un polymère de cellulose.

11. Utilisation selon la revendication 10, dans laquelle la couche de séparation comprend une première couche comprenant un polymère d'hydroxypropylméthylcellulose de faible poids moléculaire et une deuxième couche comprenant du poly(alcool vinylique).

12. Procédé pour préparer une formulation de duloxétine selon l'une quelconque des revendications 1 à 9, le procédé comprenant :
(i) la fourniture d'un noyau comprenant une quantité souhaitée de duloxétine ;
(ii) l'application d'un enrobage entérique comprenant du phtalate d'hydroxypropylméthylcellulose sur le noyau ; et
(iii) l'application d'une couche de séparation au noyau et l'application de l'enrobage entérique sur la couche de séparation,
la couche de séparation de (iii) comprenant du poly(alcool vinylique) plus, facultativement, un polymère de cellulose.

13. Procédé selon la revendication 12, dans lequel l'étape (iii) comprend les sous-étapes de :
(a) application d'une première couche comprenant un polymère d'hydroxypropylméthylcellulose de faible poids moléculaire ; et
(b) l'application d'une deuxième couche de polymère comprenant du poly(alcool vinylique).

14. Procédé selon la revendication 12 ou 13, dans lequel l'étape (ii) et/ou l'étape (iii) est/sont conduite(s) dans un appareil à lit fluidisé.

15. Procédé selon l'une quelconque des revendications 12 à 14, dans lequel l'étape (i) comprend l'application de duloxétine et d'un liant sur une bille inerte pharmaceutiquement acceptable.
